# EUROPÄISCHE PATENTSCHRIFT

(11) **EP 3 443 063 B1**
(45) Veröffentlichungstag und Bekanntmachung des Hinweises auf die Patenterteilung: **17.06.2020**
(21) Anmeldenummer: 17714675.0
(22) Anmeldetag: 24.03.2017
(51) Int. Cl.: C12M 1/00

(54) **VERFAHREN ZUM HYGIENISIEREN VON BIOMASSE**
METHOD FOR SANITIZING BIOMASS
PROCÉDÉ D'HYGIÉNISATION DE BIOMASSE

(30) Priorität: 12.04.2016 DE 102016106746
(43) Veröffentlichungstag der Anmeldung: 20.02.2019
(73) Patentinhaber: CEBCON Technologies GmbH, 22607 Hamburg (DE)
(72) Erfinder: DETZEL, Valery, 22335 Hamburg (DE)
(74) Vertreter: Hauck Patentanwaltspartnerschaft mbB
(86) Internationale Anmeldenummer: PCT/EP2017/057106
(87) Internationale Veröffentlichungsnummer: WO 2017/178212

(56) Entgegenhaltungen:
- EP-A2- 0 004 081
- WO-A1-00/52405
- DE-A1- 10 339 297
- DE-A1- 10 343 415
- DE-A1- 10 352 764
- DE-A1-102013 012 289
- DE-U1- 29 707 180
- KR-A- 20070 053 178

## Beschreibung

Die Erfindung betrifft ein Verfahren zum Hygienisieren von Biomasse.

Bei der Hygienisierung geht es um Tier- und Humanhygiene (= Abtötung von Human- und Tierpathogenen) sowie um Phytohygiene (Abtötung von Phytopathogenen, Quarantäneschädlingen und Pflanzenschädlinge, Pilze und Viren, und Unkrautsamen). Ziel der Hygienisierung ist, Mikroorganismen mit pathogener Wirkung auf Pflanzen, Tiere und Menschen in Biomassen so weit abzutöten, dass nur noch ein minimales Risiko einer Übertragung von Krankheiten besteht. Durch eine Hygienisierung dieser Stoffe wird die seuchenhygienische Unbedenklichkeit erreicht.

Im Hinblick auf viele Verwendungen müssen Biomassen einer vorherigen Hygienisierung unterzogen werden. Bei der Hygienisierung werden schädliche Keime bzw. Mikroorganismen abgetötet. Beispiele hierfür sind die Verwendung von Hühnerkot oder Gülle als Düngemittel oder die Verwendung von Abfällen der Agrar-, Holz- und Forstwirtschaft als Einstreu, als Gärsubstrat für Biogasanlagen oder zu anderen Zwecken, bei denen es auf geringe Keimbelastungen ankommt.

Die Hygienisierung wird durchgeführt, indem die Biomasse über eine Zeitspanne von beispielsweise mindestens einer Stunde einer erhöhten Temperatur von beispielsweise 70°C ausgesetzt wird. Die Bedingungen für eine Hygienisierung von für nicht für den menschlichen Verzehr bestimmte tierische Nebenprodukte sind in der Verordnung (EG) Nr. 1069/2009 vom 21. Oktober 2009 angegeben.

Bekannt ist, die Hygienisierung in einem von außen beheizten Rührbehälter durchzuführen. Die Hygienisierung erfolgt chargenweise, damit keine Rückvermischung erfolgt und nicht hygienisierte Teile der Biomasse weiterverarbeitet werden. Das Rühren ist energieintensiv und die gleichmäßige Erhitzung der Biomasse ist schwer zu erreichen. Der Chargenbetrieb erlaubt nur verhältnismäßig geringe Mengendurchsätze.

Die DE 103 43 415 A1 beschreibt ein Verfahren zur Hygienisierung fließfähiger Biomasse mittels eines Mehrphasenmedium-Wärmetauschers, der mehrere horizontal angeordnete Transportrohre umfasst, die außerhalb eines Gehäuses durch Umlenkelemente miteinander verbunden sind. Durch einen Einlass unten am Gehäuse und einen Auslass oben am Gehäuse wird Wasser oder ein anderes Wärmemedium im Gehäuse zwischen den Transportrohren hindurchgeführt.

Die KR 2007 0053178 A beschreibt ein Verfahren zum Behandeln von Abwasserschlamm, bei dem der Schlamm auf eine oberhalb einer Heizplatte angeordnete Drehscheibe aufgesprüht wird, so dass Wasser verdampft, Wasserdampf über zentrale Rohrleitungen abgezogen wird und die Trockenmasse über eine Ausgabevorrichtung von der Drehscheibe abgegeben werden kann.

Die DE 103 39 297 A1 beschreibt eine Stabilisatorvorrichtung für die thermische Sterilisation von einer kontinuierlich in einem Bioreaktor anfallenden pumpfähigen Biomasse. Die Biomasse wird mittels einer Pumpe durch einen Stabilisator aus mehreren lösbar verbundenen, hintereinandergeschalteten und mit einer Heißwasser-Mantelheizung versehenen Rohren hindurchgepumpt.

Die zuvor beschriebenen Vorrichtungen sind nur für pumpfähige Biomassen geeignet und insbesondere nicht für zum Hygienisieren fester, pastöser oder anderer nicht pumpfähiger Biomassen verwendet werden.

DE 103 52 764 A1 beschreibt einen Schachtvorwärmer zum Vorwärmen von stückigem Material, bei dem der Schacht mittels einer Einrichtung zum Befüllen mittig beschickt wird sich dabei ein Schüttkegel ausbildet. Eine im Schacht angeordnete Brücke zur Zuführung von heißen Gasen, die das Material von unten nach oben durchströmen, ist vorteilhafter Weise entsprechend den sich ausbildenden Schüttkegel ausgebildet.

Davon ausgehend liegt der Erfindung die Aufgabe zugrunde, ein energetisch, hinsichtlich der gleichmäßigen Erhitzung und der Mengendurchsätze verbessertes Verfahren zum Hygienisieren von Biomasse zur Verfügung zu stellen.

Die Aufgabe wird durch ein Verfahren mit den Merkmalen von Anspruch 1 gelöst. Vorteilhafte Ausgestaltungen des Verfahrens sind in Unteransprüchen angegeben.

Bei dem erfindungsgemäßen Verfahren zum Hygienisieren von Biomasse wird die Biomasse einem als Schachtkühler ausgebildeten Schachterhitzer zugeführt, der oben eine Aufgabeeinrichtung zum gleichmäßigen Verteilen des zu hygienisierenden Materials auf verschiedene Schächte des Schachterhitzers aufweist, und die Biomasse in dem Schachterhitzer erhitzt, indem dem Schachterhitzer ein erhitztes Heizmedium zugeführt wird.

Bei dem erfindungsgemäßen Verfahren wird ein Schachtkühler, der herkömmlicherweise zum Kühlen von Pellets oder anderen festen Granulaten aus kleinstückigem Material organischen und/oder pflanzlichen Ursprunges herangezogen wird, für das Hygienisieren von Biomasse verwendet. Aufgrund seiner erfindungsgemäßen Verwendung für die Hygienisierung wird der Schachtkühler auch als "Schachterhitzer" bezeichnet. Hierfür wird dem Schachterhitzer anstatt eines Kühlmediums ein Heizmedium zugeführt. Das Heizmedium wird erhitzt dem Kühlmediumeintritt des Schachterhitzers zugeführt und abgekühlt aus dem Kühlmediumaustritt des Schachterhitzers abgeführt. Dem Kühlmediumeintritt wird es mit einer Temperatur zugeführt, die mindestens gleich der Temperatur zum Hygienisieren der Biomasse ist. Vorzugsweise wird gemäß dem Verfahren schüttfähige Biomasse hygienisiert. Vorteilhaft ist der besonders gute und gleichmäßige Wärmeübergang vom Heizmedium auf die Biomasse im Schachterhitzer aufgrund der großen wärmeübertragenden Flächen, die Kontakt mit der Biomasse haben. Weiterhin vorteilhaft ist, dass der Schachterhitzer ein enges Verweilzeitspektrum hat, so dass das zu hygienisierende Material gleichmäßig durch die Schächte hindurch wandern kann. Infolgedessen kann mittels des Schachterhitzers eine kontinuierliche Hygienisierung

erfolgen, ohne dass aufgrund von Rückvermischung oder Kurzschlussströmung noch nicht vollständig hygienisiertes Material den Schachterhitzer verlässt. Bei der kontinuierlichen Arbeitsweise wird die zu hygienisierende Biomasse kontinuierlich zu- und abgeführt. Hierdurch wird eine besonders wirtschaftliche Arbeitsweise erreicht. Grundsätzlich ist es aber auch möglich, den Schachterhitzer absatzweise zur Hygienisierung von Biomasse einzusetzen, indem der Schachterhitzer zunächst mit der zu hygienisierenden Biomasse befüllt wird, dann verschlossen und nach einer bestimmten Zeitspanne das hygienisierte Material entnommen wird. Mittels der Aufgabeeinrichtung ist der Schachterhitzer besonders effektiv betreibar.

Gemäß einer vorteilhaften Ausführungsart der Erfindung ist die Biomasse tierischer Herkunft und/oder pflanzlicher Herkunft.

Gemäß einer weiteren Ausführungsart ist die Biomasse ausgewählt aus mindestens einem der folgenden Materialien: Hühnerkot, Gülle oder andere Tierexkremente, Schlachtabfälle, Tierkadaver oder andere Tierabfälle, Lebensmittelreste, Küchenabfälle oder andere Hausmüllbestandteile, Säge- oder Hobelspäne, Hackschnitzel, Holzschreddergut oder andere Nebenprodukte oder Abfälle der Holz- und Forstwirtschaft, Stroh, Sonnenblumenschalen, Olivenkerne, Olivenpresstrester, Reisschalen oder andere biogene Reststoffe der Agrarwirtschaft oder Lebensmittelindustrie, Gärsubstrate (Fütterungssubstrate) zum Einspeisen in Biogasanlagen oder Gärreste aus Biogasanlagen.

Gemäß einer bevorzugten Ausführungsart wird die Biomasse dem Schachterhitzer in schüttfähiger Form, vorzugsweise in fester Form, vorzugsweise in Form von Pellets oder anderen festen Granulaten aus kleinstückigem Material zugeführt. Besonders vorteilhaft ist der Schachterhitzer für die Erhitzung der Biomasse in Form von Pellets oder anderen festen Granulaten geeignet.

Gemäß einer weiteren Ausführungsart wird die Biomasse in dem Schachterhitzer auf eine Temperatur von mindestens 110°C, vorzugsweise von mindestens 90°C, vorzugsweise von mindestens 80°C, vorzugsweise von mindestens 75°C, vorzugsweise von mindestens 70°C erhitzt und/oder wird die Biomasse im Schachterhitzer über eine Zeitspanne von mindestens 1,5 Stunden, vorzugsweise von mindestens 1,1 Stunden, vorzugsweise von mindestens 1 Stunde erhitzt.

Gemäß einer weiteren Ausführungsart wird als Heizmedium für das Erhitzen der Biomasse im Schachterhitzer Wasser oder Thermoöl verwendet.

Gemäß einer weiteren bevorzugten Ausführungsart wird zum Hygienisieren ein Schachterhitzer verwendet, der zumindest teilweise in mindestens einem Container angeordnet ist. Vorzugsweise wird ein Schachterhitzer verwendet, der vollständig in einem einzigen Container angeordnet ist. Dies erleichtert das Errichten einer Anlage zum Hygienisieren von Biomasse, insbesondere wenn die Anlage zu verschiedenen Zeiten an verschiedenen Standorten eingesetzt werden soll. Der Container ist kostengünstig auf Schiene, Straße oder Wasserweg vom Werk zum Einsatzort oder von einem früheren Einsatzort zu einem neuen Einsatzort transportierbar.

Gemäß einer weiteren Ausführungsart weist der Container die Abmessungen eines 20-Fuß- oder eines 40-Fuß-Containers oder eines anderen Standardcontainers auf. Dies ist vorteilhaft für den Transport des Containers und der Einsatz vorhandener Transportmittel. Gemäß einer bevorzugten Ausführungsart weist der Container die Eigenschaften (z.B. Stapelbarkeit, Transportierbarkeit, Verzurrung miteinander) von Standardcontainern auf. Gemäß einer weiteren Ausführungsart ist der Container eine Rahmenkonstruktion mit offenen Wänden oder einer oder mehreren geschlossenen Wänden. Die Rahmenkonstruktion weist einen Rahmen aus mehreren Rahmenteilen auf. Bei einer offenen Rahmenkonstruktion umgeben Rahmenteile Öffnungen, die nicht durch die Wände ausgefüllt sind. Bei einer geschlossenen Rahmenkonstruktion sind die Öffnungen zwischen Rahmenteilen durch Wände ausgefüllt bzw. geschlossen. Mischformen mit einer teilweise offenen und teilweise geschlossenen Rahmenkonstruktion sind ebenfalls Bestandteil der Erfindung. Vorzugsweise ist die Rahmenkonstruktion quaderförmig, wobei die Rahmenteile die Kanten des Quaders definieren.

Die Ausführung als Rahmenkonstruktion reduziert Gewicht und Kosten. Zudem begünstigt sie Montage, Betrieb und Wartung der Anlage.

Alternativ ist der Container ein herkömmlicher Standardcontainer mit geschlossenen Wänden. Gemäß einer Ausgestaltung ist der Container durch an den Rändern miteinander verbundene Wände gebildet. Gegebenenfalls sind die Wände und/oder die Verbindungen benachbarter Wände miteinander an den Ecken und/oder Kanten des Containers verstärkt.

Bei der Ausführung des Containers als Rahmenkonstruktion mit offenen Wänden bilden die Rahmenteile die Containerhülle. Bei Ausführung des Containers als Rahmenkonstruktion mit einer oder mehreren geschlossenen Wänden bilden die Rahmenteile und die diese ausfüllenden Wände die Containerhülle. Bei Ausführung des Containers als Kasten aus randseitig miteinander verbundenen Wänden bilden die Wände die Containerhülle.

Gemäß einer weiteren Ausführungsart ist mindestens ein Strukturelement des Schachterhitzers mindestens teilweise Bestandteil einer Containerhülle. Gemäß einer bevorzugten Ausgestaltung ist mindestens ein Strukturelement des Schachterhitzers Bestandteil mindestens einer Wand und/oder mindestens eines Rahmenteils des Rahmens. Gemäß einer weiteren Ausführungsart ist mindestens eine Seitenwand und/oder mindestens ein Rahmenteil des Schachterhitzers Bestandteil einer Wand und/oder eines Rahmenteils des Containers. Beispielsweise ist mindestens eine Seitenwand eines Kühlergehäuses des Schachterhitzers, die einen Raum begrenzt, indem die zu kühlende Biomasse gekühlt wird, Bestandteil einer Wand des Containers oder bildet die gesamte Wand des Containers. Vorzugsweise sind mehrere Seitenwände des Schachterhitzers zugleich Wände des Containers oder Bestandteile derselben.

Gemäß einer weiteren Ausführungsart ist der Schachterhitzer in einem Container mit vertikal ausgerichteter Längsachse untergebracht.

Gemäß einer weiteren Ausführungsart umfasst der Schachterhitzer mehrere Rohrmäander, die parallel, mit Abstand voneinander und um den halben Abstand ihrer horizontalen Rohrabschnitte höhenversetzt zueinander angeordnet sind. Die Rohrmäander sind vorzugsweise vertikal ausgerichtet. Das Material wird beim Durchlaufen durch die zwischen den Rohrmäandern gebildeten Schächte des Schachterhitzers von den horizontalen Rohrabschnitten der Rohrmäander abwechselnd in die eine und in die andere Richtung horizontal abgelenkt. Der Schachterhitzer heizt die Biomasse besonders effektiv und schonend (insbesondere unter Vermeidung von Staubbildung und hohen Abluftmengen) auf und hat einen geringen Grundflächen- und Raumbedarf.

Gemäß einer weiteren Ausführungsart sind die horizontalen Rohrabschnitte jedes Rohrmäanders durch vertikale Stege miteinander verbunden. Die vertikalen Stege verbessern die Stabilität des Rohrmäanders. Zudem bewirken sie eine für eine gleichmäßige Erhitzung vorteilhafte Führung des zu erhitzenden Materials und vergrößern die Wärmetauscherfläche.

Die Rohrmäander sind beispielsweise aus gebogenen Rohren gebildet, zwischen die Stege eingesetzt sind. Gemäß einer anderen Ausgestaltung sind die Rohrmäander und Stege gebildet aus umgeformten (z.B. mittels Tiefziehverfahren oder Innenhochdruckumformverfahren) und miteinander verbundenen Metallplatten, analog zu bekannten Flachheitskörpern aus Stahlblech.

Gemäß einer weiteren Ausführungsart weist der Schachterhitzer unten einen Austragsboden mit einstellbarem Öffnungsquerschnitt zum Regeln des Füllstands des Materials innerhalb der Schächte auf.

Mittels des Austragsbodens ist der Schachterhitzer besonders effektiv betreibbar.

Gemäß einer weiteren Ausführungsart ist der Schachterhitzer mit einer Einrichtung zum Absaugen von Ausdünstungen und Restdampf verbunden.

Gemäß einer weiteren Ausführungsart ist der Schachterhitzer mit einer Einrichtung zum Absaugen von Ausdünstungen und Restdampf verbunden und in die Einrichtung zum Absaugen eine Wärmerückgewinnung integriert. Hierdurch kann die Energieeffizienz des Verfahrens weiter verbessert werden.

Gemäß einer weiteren Ausgestaltung wird die hygienisierte Biomasse einer Wärmerückgewinnung zugeführt. Die aus der erhitzten Biomasse zurückgewonnene Wärme kann beispielsweise zum Vorheizen der Biomasse vor dem Einspeisen in den Schachterhitzer verwendet werden.

Die Erfindung wird nachfolgend anhand der anliegenden Zeichnungen eines Ausführungsbeispiels näher erläutert. In den Zeichnungen zeigen:
- Fig. 1: einen Schachterhitzer in einem ersten Vertikalschnitt;
- Fig. 2: den Schachterhitzer in einem zweiten Vertikalschnitt;
- Fig. 3: die Rohrmäander des Schachterhitzers in eine Seitenansicht;
- Fig. 4: einen Rohrmäander des Schachterhitzers in einer Vorderansicht;
- Fig. 5: einen weiteren Rohrmäander des Schachterhitzers in einer Vorderansicht;
- Fig. 6: einen Schachterhitzer in einer Übersichtszeichnung (Außenansicht);
- Fig. 7: einen Schachterhitzer in einer Übersichtszeichnung (Innenansicht).

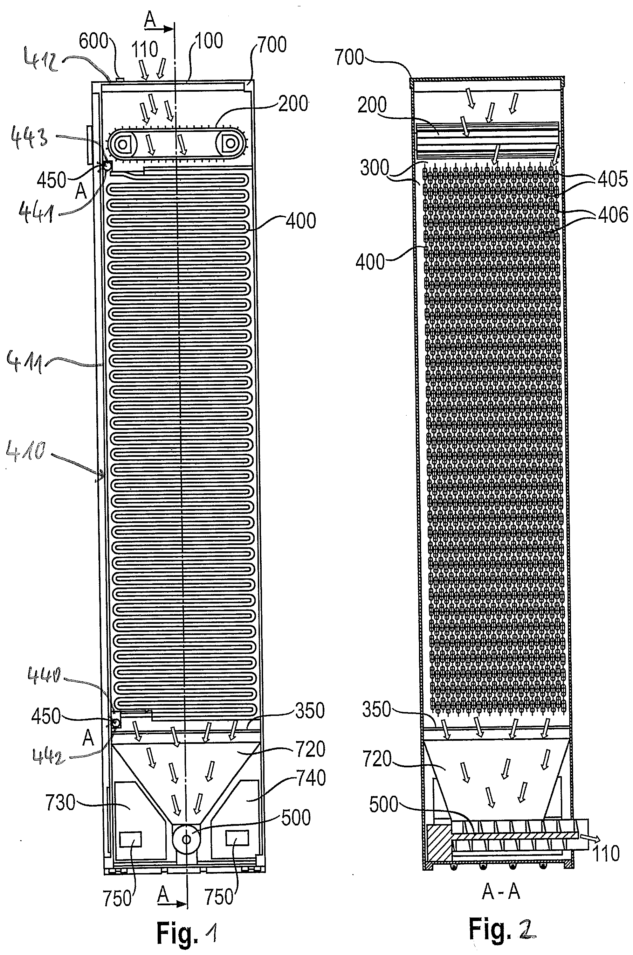

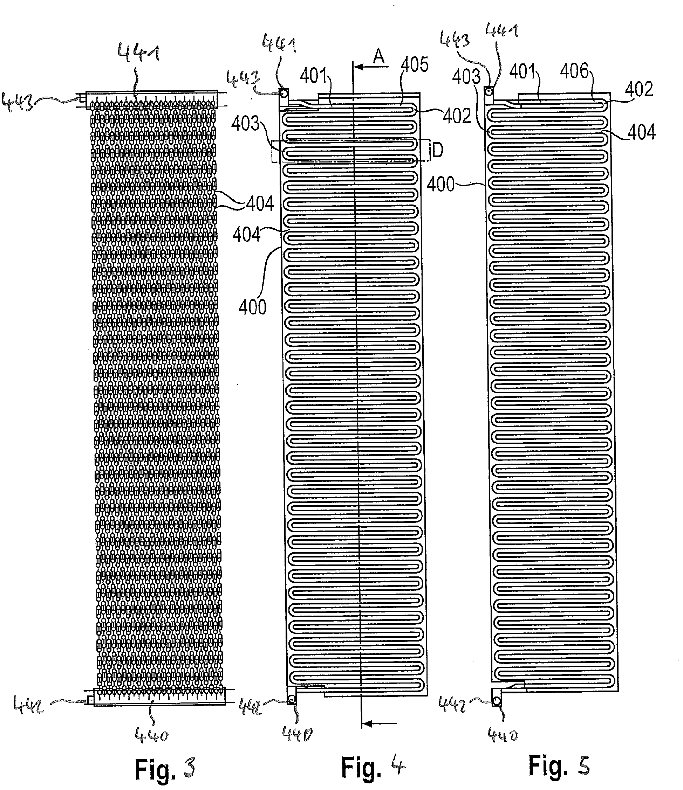

Die Erläuterung der Ausführung und Funktionsweise des Schachterhitzers 12 erfolgt anhand der Fig. 1 bis 7.

Durch die Einfüllöffnung 100 in einer Deckwand 412 eines kastenförmigen Kühlergehäuses 410 werden Pellets 110, Granulat oder anderes schüttfähiges Material aus Biomasse (nachstehend vereinfachend "Pellets" genannt) eingeführt, und mit einem unterhalb der Deckwand angeordneten Kratzkettenförderer 200 über parallele vertikale Schächte 300 innerhalb von vier Seitenwänden 411 des Kühlergehäuses 410 verteilt. Ein beweglicher Schlitzboden 350 unterhalb der Schächte 300 bleibt solange geschlossen, bis die Schächte 300 voll sind. Durch Wärmetauscherkörper 400 wird Heizmedium 450 geführt und dieses führt den Pellets 110 Wärmeenergie zu. Hierdurch werden die Pellets 110 auf z.B. 70°C erhitzt.

Die Pellets 110 rutschen stetig und gleichmäßig in den Schächten 300 nach unten, wobei ihre Geschwindigkeit durch die Spaltgröße im Schlitzboden 350 reguliert wird. Die Geschwindigkeit wird so eingestellt, dass die Verweilzeit der Pellets 110 den Schächten 300 eine Stunde beträgt.

Nach Passieren des Schlitzbodens 350 werden die erhitzten Pellets 110 durch einen Austragungstrichter 720 einer Austragungsschnecke 500 zugeführt und von dieser ausgetragen. Der Austragungstrichter 720 begrenzt unten das Kühlergehäuse 410. Die Öffnungen 600 in der Deckwand 412 dienen dem Entweichen der Ausdunstungen und des Restdampfes aus den heißen Pellets 110. Ferner ist in der Deckwand 412 eine Revisionsklappe 416 vorhanden.

In Fig. 1 bis 5 ist die Ausformung der Wärmetauscherkörper 400 dargestellt.

Die Wärmetauscherkörper 400 sind als verbundene Schlangenrohrsysteme ausgeführt. Die Wärmetauscherkörper 400 haben parallele horizontale Rohrabschnitte 401, die an den Enden durch Rohrbögen 402 miteinander verbunden sind, mit denen sie Rohrmäander 403 bilden. Ferner haben sie vertikale Stege 404 zwischen benachbarten horizontalen Rohrabschnitten 401.

Verbaut sind 2 Gruppen 405, 406 von Wärmetauscherkörpern 400, wobei die horizontalen Rohrabschnitte 401 der Wärmetauscherkörper 400 verschiedener Gruppen 405, 406 um einen halben Abstand D zwischen zwei benachbarten horizontalen Rohrabschnitten 401 eines Wärmetauscherkörpers 400 versetzt zueinander sind. Dies führt zu einer Seitwärtsbewegung der Pellets 110 in dem Schacht 300 und einer Vergrößerung der Kontaktfläche. Gleichzeitig erfolgt eine anteilige Umwälzung der Pellets 110 in dem Schacht 300, was die Gleichmäßigkeit der Erhitzung verbessert.

Jeder Rohrmäander 403 mündet unten in einem unteren Sammelrohr 440 und oben in einem oberen Sammelrohr 441. Das untere Sammelrohr 440 hat in einer Seitenwand 411 des Schachtkühlers 12 eine untere Öffnung 442, die als Kühlmediumeintritt dient. Das obere Sammelrohr 441 hat an einem oberen Ende in einer Seitenwand 411 des Schachtkühlers 12 eine obere Öffnung 443, die als Kühlmediumaustritt dient. Das Heizmedium 450 tritt durch die untere Öffnung 442 in den Schachterhitzer 12 ein und verlässt ihn durch die obere Öffnung 443.

Der Schachterhitzer ist vollständig in einem horizontal oder vertikal ausgerichteten Container untergebracht.

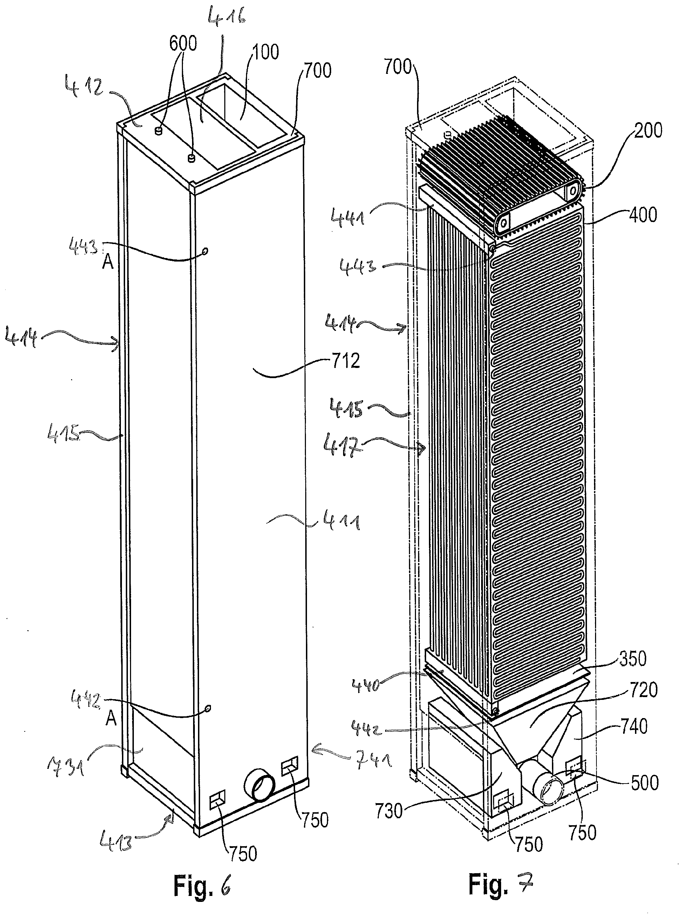

Fig. 6 und 7 zeigen einen vertikal ausgerichteten Container 700, der einen vollständigen Schachterhitzer 712 enthält. Die Seitenwände 411 und die Deckwand 412 des Kühlergehäuses 410 sind zugleich äußere Wände des Containers 700. Die Seitenwände 411 erstrecken sich vorzugsweise bis zum unteren Ende des Containers 700. Der Container 700 hat vorzugsweise unten eine Bodenwand 413. Vorzugsweise weist der Container 700 einen Rahmen 414 auf und sind die Seitenwände 411, Deckwand 412 und Bodenwand 413 jeweils randseitig in Öffnungen 417 zwischen Rahmenteilen 415 des Rahmens 414 gehalten, um einen zumindest teilweise geschlossenen Rahmen zu bilden. Durch die integrale Ausbildung der Wände 411, 412 und Rahmenteile 415 des Kühlergehäuses 410 und des Containers 700 sind tragende Strukturelemente des Containers 700 zugleich Bestandteile des Schachtkühlers 12 und wird Material und Gewicht eingespart.

Gemäß Fig. 1, 6 und 7 sind in den Freiräumen innerhalb des Containers 700 beidseitig eines Austragungstrichters 720, der die erhitzten Pellets der Austragungsschnecke 500 zuführt, Schaltschränke 730, 740 angeordnet. An den Schaltschränken 730 und 740 befinden sich Schnittstellen 750 für Energie und Daten. Dort weisen vorzugsweise die Wände des Containers 700 weitere Revisionsklappen oder Revisionsöffnungen 731, 741 auf.

### Bezugszeichenliste

- 12: Schachterhitzer
- 110: Pellets
- 200: Kratzkettenförderer
- 300: Schacht
- 350: Schlitzboden
- 400: Wärmetauscherkörper
- 401: horizontaler Rohrabschnitt
- 402: Rohrbogen
- 403: Rohrmäander
- 404: vertikaler Steg
- 405: Gruppe
- 406: Gruppe
- 410: Kühlergehäuse
- 411: Seitenwand
- 412: Deckwand
- 413: Bodenwand
- 414: Rahmen
- 415: Rahmenteil
- 416: Revisionsklappe
- 417: Öffnung
- 440: unteres Sammelrohr
- 441: oberes Sammelrohr
- 442: untere Öffnung
- 443: obere Öffnung
- 450: Heizmedium
- 500: Austragsschnecke
- 600: Öffnung
- 700: Container
- 712: Schachterhitzer
- 720: Austragungstrichter
- 730: Schaltschrank
- 731: Revisionsöffnung
- 740: Schaltschrank
- 741: Revisionsöffnung
- 750: Schnittstelle für Energie und Daten

## Patentansprüche

1. Verfahren zum Hygienisieren von Biomasse, bei dem die Biomasse einem wie ein Schachtkühler ausgebildeten Schachterhitzer zugeführt wird, der oben eine Aufgabeeinrichtung zum gleichmäßigen Verteilen des zu hygienisierenden Materials auf verschiedenen Schächte aufweist, und die Biomasse in dem Schachterhitzer erhitzt wird, indem dem Schachtkühler ein erhitztes Heizmedium zugeführt wird.

2. Verfahren nach Anspruch 1, bei dem die Biomasse tierischer Herkunft und/oder pflanzlicher Herkunft ist.

3. Verfahren nach Anspruch 2, bei dem die Biomasse ausgewählt ist aus mindestens einem der folgenden Materialien: Hühnerkot, Gülle oder andere Tierexkremente, Schlachtabfälle, Tierkadaver oder andere Tierabfälle, Lebensmittelreste, Küchenabfälle oder andere Hausmüllbestandteile, Säge- oder Hobelspäne, Hackschnitzel, Holzschreddergut oder andere Nebenprodukte oder Abfälle der Holz- und Forstwirtschaft, Stroh, Sonnenblumenschalen, Olivenkerne, Olivenpresstrester, Reisschalen oder andere biogene Reststoffe der Agrarwirtschaft oder Lebensmittelindustrie, Gärsubstrate für Biogasanlagen oder Gärreste aus Biogasanlagen.

4. Verfahren nach einem der Ansprüche 1 bis 3, bei dem dem Schachterhitzer schüttfähige Biomasse, vorzugsweise in fester Form, vorzugsweise in Form von Pellets oder anderen festen Granulaten aus kleinstückigem Material zugeführt wird.

5. Verfahren nach einem der Ansprüche 1 bis 4, bei dem die Biomasse in dem Schachterhitzer auf eine Temperatur von mindestens 70°C erhitzt wird und/oder bei dem die Biomasse im Schachtkühler über eine Zeitspanne von mindestens eine Stunde erhitzt wird.

6. Verfahren nach einem der Ansprüche 1 bis 5, bei dem als Heizmedium zum Erhitzen der Biomasse im Schachterhitzer Wasser oder Thermoöl verwendet wird.

7. Verfahren nach einem der Ansprüche 1 bis 6, bei dem der Schachterhitzer vollständig oder teilweise in mindestens einem Container angeordnet ist.

8. Verfahren nach einem der Ansprüche 1 bis 7, bei dem der Schachterhitzer zumindest teilweise in mindestens einem einzeln transportierbaren Container angeordnet ist, der eine Containerhülle aufweist, wobei mindestens ein Strukturelement des Schachterhitzers mindestens teilweise Bestandteil der Containerhülle ist.

9. Verfahren nach einem der Ansprüche 1 bis 8, bei dem der Schachterhitzer mehrere Rohrmäander umfasst, die parallel, mit Abstand voneinander und um den halben Abstand ihrer horizontalen Rohrabschnitte höhenversetzt zueinander angeordnet sind, so dass das Material beim Durchlaufen durch die zwischen den Rohrmäandern gebildeten Schächte des Schachterhitzers vor den horizontalen Rohrabschnitten der Rohrmäander abwechselnd in die eine und die andere Richtung horizontal abgelenkt wird.

10. Verfahren nach Anspruch 9, bei dem die horizontalen Rohrabschnitte jedes Rohrmäanders durch vertikale Stege miteinander verbunden sind.

11. Verfahren nach einem der Ansprüche 1 bis 10, bei dem der Schachterhitzers unten einen Austragsboden mit einstellbarem Öffnungsquerschnitt zum Regeln des Füllstandes des Materials innerhalb der Schächte aufweist.

## Claims

1. Method for sanitizing biomass, in which the biomass is fed to a shaft heater designed like a shaft cooler, which at the top has a feed apparatus for evenly distributing the material to be sanitized to the various shafts, and the biomass is heated in the shaft heater by supplying a heated heating medium to the shaft cooler.

2. The method according to claim 1, wherein the biomass is of animal origin and/or vegetable origin.

3. The method according to claim 2, wherein the biomass is selected from at least one of the following materials: Chicken dung, manure or other animal excrements, slaughtering waste, animal cadavers or other animal wastes, food wastes, kitchen wastes or other household garbage components, sawdust or wood shavings, wood chips, wood shredder material or other byproducts or waste from the timber industry and forestry, straw, sunflower shells, olive pits, olive pressing residue, rice husks or other biological residue from agriculture or the food industry, fermentation substrate for biogas plants, or fermentation residue from biogas plants.

4. The method according to one of claims 1 to 3, wherein pourable biomass, preferably in solid form, preferably in the form of pellets or other solid granules of small particulate material, is fed to the shaft cooler.

5. The method according to one of claims 1 to 4, wherein the biomass in the shaft heater is heated to a temperature of at least 70°C, and/or wherein the biomass in the shaft heater is heated over a time span of at least one hour.

6. The method according to one of claims 1 to 5, wherein water or thermal oil is used as the heating medium to heat the biomass in the shaft heater.

7. The method according to one of claims 1 to 6, wherein the shaft heater is arranged entirely or partially in at least one container.

8. The method according to one of claims 1 to 7, wherein the shaft heater is at least partially arranged in at least one individual transportable container that has a container shell, wherein at least one structural element of the shaft heater is at least partially a component of the container shell.

9. The method according to one of claims 1 to 8, wherein the shaft heater comprises a plurality of pipe meanders that are arranged parallel at a distance from each other and are offset in height by one half the distance of their horizontal pipe sections so that the material is alternately deflected horizontally in one direction and the other direction by the horizontal pipe sections of the pipe meanders while passing through the shafts of the shaft heater formed between the pipe meanders.

10. The method according to claim 9, wherein the horizontal pipe sections of each pipe meander are connected to each other by vertical bars.

11. The method according to one of claims 1 to 10, wherein the shaft heater has a discharge floor at the bottom with an adjustable opening cross-section to regulate the fill level of the material within the shafts.

## Revendications

1. Procédé pour l'hygiénisation de biomasse, dans lequel la biomasse est alimentée vers un système de chauffage de puits conçu comme un refroidisseur de puits, lequel présente un dispositif de chargement sur le haut pour la répartition homogène de la matière à hygiéniser entre différents puits, et la biomasse est chauffée dans le système de chauffage de puits en alimentant un fluide chauffant chauffé vers le refroidisseur de puits.

2. Procédé selon la revendication 1, dans lequel la biomasse est d'origine animale et/ou d'origine végétale.

3. Procédé selon la revendication 2, dans lequel la biomasse est sélectionnée parmi l'une au moins des matières suivantes : fientes, lisier ou autres excréments d'animaux, déchets d'abattoir, cadavres d'animaux ou autres déchets animaux, restes de nourriture, déchets de cuisine ou autres composants de déchets ménagers, sciure ou copeaux de bois, éclats de bois, matières de bois broyées ou autres produits dérivés ou déchets de l'industrie du bois et de la sylviculture, paille, coques de tournesol, noyaux d'olive, margines d'olive, paille de riz ou autres résidus biogènes du secteur agricole ou de l'industrie agroalimentaire, substrats de fermentation pour installations de biogaz ou résidus de fermentation provenant d'installations de biogaz.

4. Procédé selon l'une des revendications 1 à 3, dans lequel le système de chauffage de puits reçoit de la biomasse en vrac, de préférence sous forme solide, de préférence sous forme de pellets ou d'autres granulats solides constitués de matière réduite en morceaux.

5. Procédé selon l'une des revendications 1 à 4, dans lequel la biomasse dans le système de chauffage de puits est chauffée à une température d'au moins 70°C et/ou dans lequel la biomasse dans le refroidisseur de puits est chauffée pendant une durée d'au moins une heure.

6. Procédé selon l'une des revendications 1 à 5, dans lequel de l'eau ou de l'huile thermique est utilisée comme fluide chauffant pour chauffer la biomasse dans le système de chauffage de puits.

7. Procédé selon l'une des revendications 1 à 6, dans lequel le système de chauffage de puits est disposé entièrement ou partiellement dans au moins un conteneur.

8. Procédé selon l'une des revendications 1 à 7, dans lequel le système de chauffage de puits est disposé au moins partiellement dans au moins un conteneur apte à être transporté séparément, lequel présente une enveloppe de conteneur, dans lequel au moins un élément structurel du système de chauffage de puits fait au moins partiellement partie de l'enveloppe de conteneur.

9. Procédé selon l'une des revendications 1 à 8, dans lequel le système de chauffage de puits comporte plusieurs méandres de tuyau, lesquels sont disposés parallèlement et à une distance les uns des autres, tout en étant décalés en hauteur les uns par rapport aux autres selon la moitié de la distance entre leurs sections de tuyau horizontales, de telle façon que la matière est déviée horizontalement, alternativement dans l'une ou l'autre direction, lors du passage à travers les puits du système de chauffage de puits formés entre les méandres de tuyau, en amont des sections de tuyau horizontales des méandres de tuyau.

10. Procédé selon la revendication 9, dans lequel les sections de tuyau horizontales de chaque méandre de tuyau sont reliées entre elles par des branches verticales.

11. Procédé selon l'une des revendications 1 à 10, dans lequel le système de chauffage de puits présente un fond de déchargement sur le bas, avec une section transversale d'ouverture réglable permettant de régler le niveau de remplissage de la matière à l'intérieur des puits.
